# EUROPEAN PATENT APPLICATION

(11) **EP 1 992 943 A1**
(43) Date of publication of application: **19.11.2008**
(21) Application number: 07715077.9
(22) Date of filing: 22.02.2007
(51) Int. Cl.: G01N 31/22, G01N 21/78, G01N 31/00, G01N 33/84

(54) **METHOD OF DETERMINING METAL BY COLORIMETRY AND DETERMINATION REAGENT**

(30) Priority: 23.02.2006 JP 2006084154
(71) Applicant: SHINO-TEST CORPORATION, Chiyoda-ku, Tokyo 101-8410 (JP)
(72) Inventor: HIGURASHI, Kazuhiko, Shino-Test Corporation, Sagamihara-shi, Kanagawa 2290011 (JP); IIZUKA, Naomi, Shino-Test Corporation, Sagamihara-shi, Kanagawa 2290011 (JP)
(74) Representative: Betten & Resch
(86) International application number: PCT/JP2007/053852
(87) International publication number: WO 2007/097468

(57) **Abstract**

The present invention is a method for colorimetrically determining a metal in a sample using a chelating coloring agent, **characterized by** coexistence of a masking agent for iron, copper or nickel. Also, the present invention is a reagent for colorimetrically determining a metal in a sample using a chelating coloring agent, **characterized by** inclusion of a masking agent for iron, copper and/or nickel. Further, the present invention is a masking agent for iron, copper and/or nickel and a method for reducing positive errors due to iron, copper or nickel contained in a sample.

## Description

### TECHNICAL FIELD

The present invention relates to methods and reagents for determining metals.

In particular, the present invention is useful in the field of chemistry, life science, analytical science, clinical laboratory testing and the like.

### BACKGROUND ART

Various metals exist in an organism and exert a diversity of functions. As such, determination of metals in biological samples is recognized as important. For example, zinc is one of metal elements that are essential for vital functions, which is widely distributed in an organism and it is known that deficiency of zinc may cause such symptoms as dysgenesis and dysgeusia. Thus, determination of zinc in a sample is an examination essential for diagnosis and treatment of such pathologies.

Methods used for determination of this metal include atomic absorption spectrometry, inductively coupled plasma (ICP) emission spectrometry and colorimetry. Among them, examples of chelating coloring agents used for colorimetry include sodium 2-(5-bromo-2-pyridylazo)-5-(N-propyl-N-sulfopropylamino)phenol (sometimes abbreviated as 5-Br-PAPS hereinafter) and sodium 2-(5-nitro-2-pyridylazo)-5-(N-propyl-N-sulfopropylamino)phenol (sometimes abbreviated as nitro-PAPS hereinafter).

These chelating coloring agents are known to usually react with multiple metals. As such, when a particular metal is to be determined, there has been a drawback that metals other than that particular metal to be determined may be included from biological samples, reagents for determination, equipment and the like to allow a chelating coloring agent to react with such included metals to introduce positive errors into measurements of the metal to be determined. In determination of a metal using a chelating coloring agent, therefore, a masking agent needs to be used for masking metals other than the metal to be determined.

When zinc is determined using 5-Br-PAPS, for example, since 5-Br-PAPS reacts with metals other than zinc, such as iron, copper and nickel, a masking agent needs to be used for masking iron, copper and nickel (see, for example, Japanese Unexamined Patent Publication No. 1985-120249). Conventionally, examples of known masking agents used for iron include citric acid, sodium fluoride and nitriloacetic acid, examples of known masking agents used for copper include salicyl aldoxime, dithiocarboxy glycine and dithiocarboxy sarcosine and examples of known masking agents used for nickel include dimethyl glyoxime.

Among these masking agents, however, some will mask zinc as a metal to be determined and some are unstable.

Incidentally, many metals exist in organisms by bonding with biomacromolecules such as proteins and exert various functions. As such, when determining a metal in a biological sample, pretreatment was needed for liberating metals from proteins and removing the proteins. Also, in prior art pretreatment, deproteinization is needed to be carried out in which a deproteinizing agent such as trichloroacetic acid is added to a sample to liberate metals from proteins and then precipitated proteins are centrifuged for separation and removal.

Such deproteinization is, however, cumbersome, time-consuming and insufficient in recovery and reproducibility of samples.

### DISCLOSURE OF THE INVENTION

It is thus an object of the present invention to provide a method for determination and a reagent for determination capable of accurately determining concentration of a metal in a sample by coexistence or inclusion of a masking agent for metals other than the metal to be determined, namely iron, copper and/or nickel.

Also, it is another object of the present invention to provide a masking agent for iron, copper and/or nickel and to provide a method for reducing positive errors due to iron, copper or nickel contained in a sample.

Further, it is another object of the present invention to provide a method for determination and a reagent for determination in which deproteinization of a sample is unnecessary in determining a metal in the sample.

As a result of keen examination for the purpose of attaining the above objects, the present inventors have found that, in a method and reagent for colorimetrically determining a metal in a sample using a chelating coloring agent, the concentration of the metal in the sample can accurately be determined by coexistence or inclusion of a masking agent for iron, copper and/or nickel to accomplish the present invention.

Specifically, the present invention provides:
(1) A method for colorimetrically determining a metal in a sample using a chelating coloring agent, characterized by coexistence of a masking agent for iron, copper and/or nickel.
(2) The method for colorimetrically determining a metal in a sample according to (1) above, wherein the chelating coloring agent is 2-(5-bromo-2-pyridylazo)-5-(N-propyl-N-3-sulfopropylamino)phenol or a salt thereof.
(3) The method for colorimetrically determining a metal in a sample according to (1) above, wherein the chelating coloring agent is 2-(5-nitro-2-pyridylazo)-5-(N-propyl-N-3-sulfopropylamino)phenol or a salt thereof.
(4) The method for colorimetrically determining a metal in a sample according to any one of (1) to (3) above, wherein a surface active agent is used.
(5) The method for colorimetrically determining a metal in a sample according to any one of (2) to (4) above, wherein deproteinization of the sample is unnecessary.
(6) The method for colorimetrically determining a metal in a sample according to any one of (1) to (5) above, wherein the masking agent for iron, copper and/or nickel is one or more selected from bis-tris propane, TAPS, TAPSO, TES, tricine, trishydroxymethylamino methane or bicine.
(7) The method for colorimetrically determining a metal in a sample according to any one of (1) to (5) above, wherein the masking agent for iron, copper and/or nickel is a compound represented by the general formula (I) : 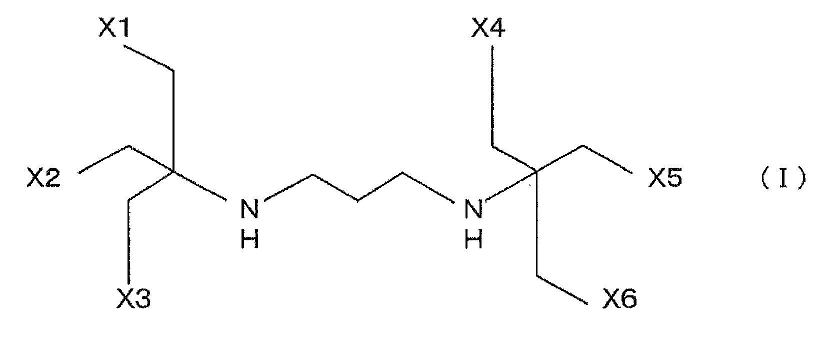 wherein X1 to X6 are respectively a hydrogen atom or an unsubstituted or substituted hydroxyl, carboxyl, SH or amino group.
(8) The method for colorimetrically determining a metal in a sample according to any one of (1) to (7) above, wherein the metal in a sample to be colorimetrically determined is zinc.
(9) The method for colorimetrically determining a metal in a sample according to any one of (1) to (8) above, wherein positive errors due to iron, copper or nickel contained in the sample can be reduced.
(10) A method for colorimetrically determining zinc in a sample using 2-(5-bromo-2-pyridylazo)-5-(N-propyl-N-3-sulfopropylamino)phenol or a salt thereof, wherein deproteinization of the sample is unnecessary.
(11) A reagent for colorimetrically determining a metal in a sample using a chelating coloring agent, characterized by inclusion of a masking agent for iron, copper and/or nickel.
(12) The reagent for colorimetrically determining a metal in a sample according to (11) above, wherein the chelating coloring agent is 2-(5-bromo-2-pyridylazo)-5-(N-propyl-N-3-sulfopropylamino)phenol or a salt thereof.
(13) The reagent for colorimetrically determining a metal in a sample according to (11) above, wherein the chelating coloring agent is 2-(5-nitro-2-pyridylazo)-5-(N-propyl-N-3-sulfopropylamino)phenol or a salt thereof.
(14) The reagent for colorimetrically determining a metal in a sample according to any one of (11) to (13) above, further containing a surface active agent.
(15) The reagent for colorimetrically determining a metal in a sample according to any one of (12) to (14) above, wherein deproteinization of the sample is unnecessary.
(16) The reagent for colorimetrically determining a metal in a sample according to any one of (11) to (15) above, wherein the masking agent for iron, copper and/or nickel is one selected from bis-tris propane, TAPS, TAPSO, TES, tricine, trishydroxymethylamino methane or bicine.
(17) The reagent for colorimetrically determining a metal in a sample according to any one of (11) to (15) above, wherein the masking agent for iron, copper and/or nickel is a compound represented by the general formula (I) : 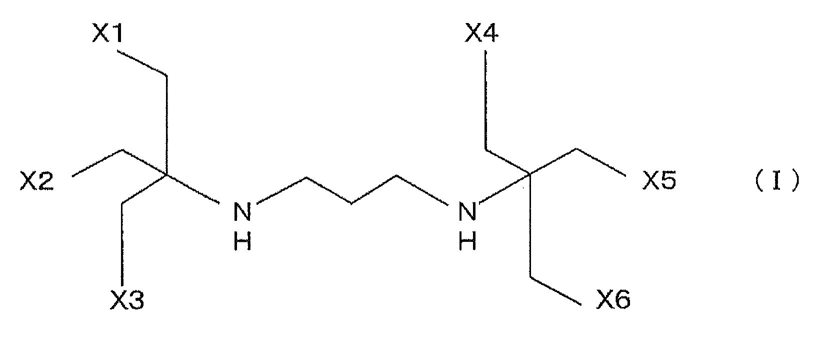 wherein X1 to X6 are respectively a hydrogen atom or an unsubstituted or substituted hydroxyl, carboxyl, SH or amino group.
(18) The reagent for colorimetrically determining a metal in a sample according to any one of (11) to (17) above, wherein positive errors due to iron, copper or nickel contained in the sample can be reduced.
(19) A masking agent for iron, copper and/or nickel, comprising bis-tris propane, TAPS, TAPSO, TES, tricine, trishydroxymethylamino methane or bicine.
(20) A masking agent for iron, copper and/or nickel, comprising a compound represented by the general formula (I): 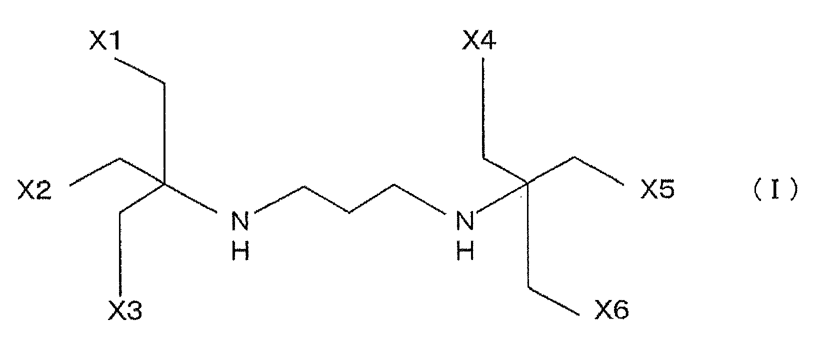 wherein X1 to X6 are respectively a hydrogen atom or an unsubstituted or substituted hydroxyl, carboxyl, SH or amino group.
(21) A method for reducing positive errors due to iron, copper or nickel contained in a sample, by coexistence of a masking agent for iron, copper and/or nickel, in a method for colorimetrically determining a metal in the sample using a chelating coloring agent.
(22) The method for reducing positive errors due to iron, copper or nickel contained in a sample according to (21) above, wherein the chelating coloring agent is 2-(5-bromo-2-pyridylazo)-5-(N-propyl-N-3-sulfopropylamino)phenol or a salt thereof.
(23) The method for reducing positive errors due to iron, copper or nickel contained in a sample according to (21) above, wherein the chelating coloring agent is 2-(5-nitro-2-pyridylazo)-5-(N-propyl-N-3-sulfopropylamino)phenol or a salt thereof.
(24) The method for reducing positive errors due to iron, copper or nickel contained in a sample according to any one of (21) to (23) above, wherein the masking agent for iron, copper and/or nickel is one selected from bis-tris propane, TAPS, TAPSO, TES, tricine, trishydroxymethylamino methane or bicine.
(25) The method for reducing positive errors due to iron, copper or nickel contained in a sample according to any one of (21) to (24) above, wherein the masking agent for iron, copper and/or nickel is a compound represented by the general formula (I): 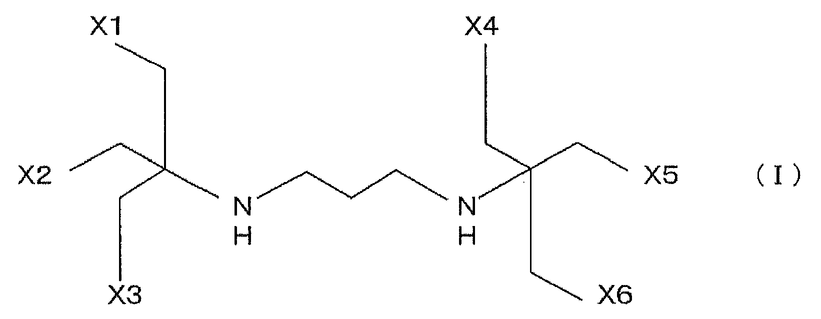
wherein X1 to X6 are respectively a hydrogen atom or an unsubstituted or substituted hydroxyl, carboxyl, SH or amino group.

According to the present invention, in colorimetrically determining a metal in a sample using a chelating coloring agent, by coexistence or inclusion of a masking agent for metals other than the metal to be determined, namely iron, copper and/or nickel, the concentration of the metal to be determined can accurately be determined even if iron, copper or nickel is included in the sample.

Also, according to the present invention, in determining a metal in a sample using a chelating coloring agent, deproteinization of the sample is unnecessary.

### BEST MODE FOR CARRYING OUT THE INVENTION

The present invention comprises a method for colorimetrically determining a metal in a sample using a chelating coloring agent, characterized by coexistence of a masking agent for iron, copper or nickel; a reagent for colorimetrically determining a metal in a sample using a chelating coloring agent, characterized by inclusion of a masking agent for iron, copper or nickel; a masking agent for iron, copper and/or nickel; and a method for reducing positive errors due to iron, copper or nickel contained in a sample.

### (1) Chelating coloring agent

According to the present invention, chelating coloring agents are used for colorimetrically determining metals in samples.

Herein, a chelating coloring agent means a reagent usable in colorimetric determination of metals which, in determining a metal other than iron, copper or nickel (metal to be determined), causes a variation in hue or absorption wavelength by contacting and bonding with the metal to be determined.

The present invention is also suitable with a chelating coloring agent that is reactive with iron, copper or nickel included in a sample and is likely to produce positive errors in measurements of a metal to be determined.

Examples of chelating coloring agents include 2-(5-bromo-2-pyridylazo)-5-(N-propyl-N-3-sulfopropylamino)phenol (sometimes abbreviated as 5-Br-PAPS hereinafter), 2-(5-nitro-2-pyridylazo)-5-(N-propyl-N-3-sulfopropylamino)phenol (sometimes abbreviated as nitro-PAPS hereinafter), 2-(5-bromo-2-pyridylazo)-5-(N-propyl-N-3-sulfopropylamino)aniline (sometimes abbreviated as 5-Br-PSAA hereinafter), Bismuthiol II, BPA, Calcein, Calcein Blue, chromotropic acid, Cu-PAN, diantipyrylmethane, murexide, nitroso-PSAP, o-phenanthroline, PAN, PAR, PDTS, PR, SATP, Tiron, TPPS, TPTZ, XO or Zincon.

According to the present invention, the chelating coloring agent is preferably 5-Br-PAPS or nitro-PAPS.

Also, the concentration of a chelating coloring agent depends on the concentration of a metal to be determined and needs to be correspondingly higher when the concentration of the metal to be determined is higher. Typically, it is preferably within the range of 10 to 500 µM and particularly preferably within the range of 10 to 50 µM in a reaction solution for determination after mixing with a sample and reagent for determination.

### (2) Masking Agent for Iron, Copper and/or Nickel

According to the present invention, colorimetric determination of a metal in a sample is made by coexistence or inclusion of a masking agent for iron, copper and/or nickel.

Herein, examples of masking agents for iron, copper and/or nickel include bis-tris propane, Bis-Tris, TAPS, TAPSO, TES, tricine, trishydroxymethylamino methane, BES or bicine. Also, according to the present invention, it is preferable to use, as a masking agent for iron, copper and/or nickel, bis-tris propane, TAPS, TAPSO, TES, tricine, trishydroxymethylamino methane or bicine.

According to the present invention, it is preferable to use a compound represented by the general formula (I): 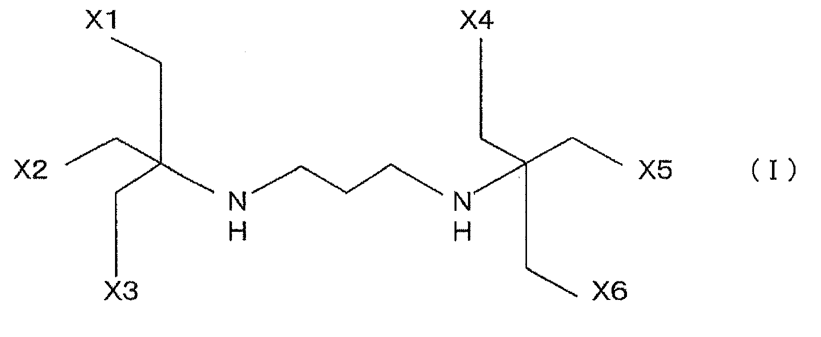 wherein X1 to X6 are respectively a hydrogen atom or an unsubstituted or substituted hydroxyl, carboxyl, SH or amino group, that is capable of masking iron, copper and nickel.

Herein, as a compound represented by the general formula (I), bis-tris propane may be mentioned for example.

Also, the concentration of a masking agent for iron, copper and/or nickel depends on the concentration of iron, copper or nickel in a sample and needs to be correspondingly higher when the concentration of iron, copper or nickel is higher. Typically, it is preferably within the range of 5 to 150 mM and particularly preferably within the range of 5 to 50 mM in a reaction solution for determination after mixing with a sample and reagent for determination. Although a masking agent may be contained at a concentration higher than 150 mM, sufficient effects may be obtained at or below such an amount.

Also, when the method for determination and the reagent for determination according to the present invention are a one-step method (one-reagent system), the concentration of a masking agent may be within the above range and when they are a two-step method (two-reagent system), a masking agent may be included in a first or second reagent so that, when a sample and the first reagent are mixed at a ratio in amount for determining a metal in the sample and when the sample and the first and second reagents are mixed at a ratio in amount for determining a metal in the sample, the concentration of the masking agent in a reaction solution for determination after mixing may be within the above range.

Also, a masking agent may be included in both first and second reagents, as long as the concentration of the masking agent in a reaction solution for determination after mixing is within the above range.

This is also true when the method for determination and the reagent for determination are a multi-step method (three or more reagents).

### (3) Surface Active Agent

According to the present invention, metals in samples may be colorimetrically determined by coexistence or inclusion of surface active agents. Surface active agents to coexist or be contained are not particularly limited in kind, and any of nonionic surface active agents, anionic surface active agents, cationic surface active agents or amphoteric surface active agents may be used for example.

Also, according to the present invention, it is preferable to use nonionic surface active agents.

Herein, examples of nonionic surface active agents include:
(a) polyoxyalkylene ether compounds such as polyoxyethylene alkyl ethers, polyoxypropylene alkyl ethers, polyoxypropylene alkylphenyl ethers, polyoxyethylene polystyrylphenyl ethers or polyoxyethylene polyoxypropylene glycols;
(b) polyhydroxy alcohol partial ester compounds, such as glycerin fatty acid partial esters, sorbitan fatty acid partial esters, pentaerythritol fatty acid partial esters, propylene glycol mono fatty acid esters or sucrose fatty acid partial esters;
(c) polyoxyethylated polyhydroxy alcohol fatty acid esters, such as polyoxyethylene sorbitan fatty acid partial esters, polyoxyethylene sorbitol fatty acid partial esters, polyoxyethylene glycerin fatty acid partial esters, polyethylene glycol fatty acid esters, polyglycerin fatty acid partial esters or polyoxyethylated castor oil; and
(d) amide or amine compounds, such as fatty acid diethanolamide, N,N-bis-2-hydroxyalkylamine, polyoxyethylene alkylamine, triethanolamine fatty acid esters or trialkylamine oxides.

According to the present invention, surface active agents may used alone or in combination of two or more.

Also the concentration of a surface active agent is preferably within the range of 0.01 to 10% and particularly preferably within the range of 0.1 to 5% in a reaction solution for determination after mixing with a sample and reagent for determination.

Also, when the method for determination and the reagent for determination according to the present invention are a one-step method (one-reagent system), the concentration of a surface active agent may be within the above range and when they are a two-step method (two-reagent system), a surface active agent may be included in a first or second reagent so that, when a sample and the first reagent are mixed at a ratio in amount for determining a metal in a sample and when the sample and the first and second reagents are mixed at a ratio in amount for determining a metal in the sample, the concentration of the surface active agent in a reaction solution for determination after mixing may be within the above range.

Also, a surface active agent may preferably be included in both first and second reagents, as long as the concentration of the surface active agent in a reaction solution for determination after mixing is within the above range.

This is also true when the method for determination and the reagent for determination are a multi-step method (three or more reagents).

### (4) Protein denaturant

According to the present invention, metals in samples may be colorimetrically determined by coexistence or inclusion of protein denaturants.

Examples of protein denaturants to coexist or be contained according to the present invention include surface active agents, such as sodium dodecylsulfate (SDS) and known protein denaturants such as urea and guanidine hydrochloride.

Also, the concentration of a protein denaturant is preferably within the range of 0.2 to 6 M and particularly preferably within the range of 0.4 to 3 M in a reaction solution for determination after mixing with a sample and reagent for determination. Although a protein denaturant may be contained at a concentration higher than 6 M, sufficient effects may be obtained at or below such an amount.

According to the present invention, protein denaturants may be used alone or in combination of two or more.

Also, when the method for determination and the reagent for determination according to the present invention are a one-step method (one-reagent system), the concentration of a protein denaturant may be within the above range and when they are a two-step method (two-reagent system), a protein denaturant may be included in a first or second reagent so that, when a sample and the first reagent are mixed at a ratio in amount for determining a metal in a sample and when the sample and the first and second reagents are mixed at a ratio in amount for determining a metal in the sample, the concentration of the protein denaturant in a reaction solution for determination after mixing may be within the above range.

Also, a protein denaturant may preferably be included in both first and second reagents, as long as the concentration of the protein denaturant in a reaction solution for determination after mixing is within the above range.

This is also true when the method for determination and the reagent for determination are a multi-step method (three or more reagents).

### (5) Other Components for Determination

According to the present invention, in addition to the components described above, known preservatives, stabilizers or the like may coexist or be contained, if necessary.

### (6) pH when Determining Metal

According to the present invention, pH when determining metals in samples may vary according to the chelating coloring agent used for the determination and, therefore, may appropriately be set at pH when determining metals. It is typically within the range of pH 5 to 11.

Also, when determining zinc in a sample, it is preferably at pH 7 or higher, and particularly preferably, within the range of pH 8 to 10.

Also, as buffers for bringing pH within the above range, conventionally known buffers having buffer capacity within the above pH range may appropriately be used.

Examples of buffers that may be used as such include carbonic acid, boric acid, phosphoric acid, trishydroxymethylamino methane, imidazole, glycylglycine, MES, Bis-Tris, ADA, ACES, bis-tris propane, PIPES, MOPSO, MOPS, BES, HEPES, TES, DIPSO, TAPSO, POPSO, HEPPS, HEPPSO, tricine, bicine, TAPS, CHES, CAPSO or CAPS or salts thereof.

### (7) Constitution of Reagent and the Like and Concentrations of Components and the Like

The method for determination and the reagent for determination according to the present invention may be implemented and constituted in one-step methods (one-reagent systems) or may be implemented and constituted in multi-step methods such as two-step methods (two-reagent systems).

Also, when the method for determination and the reagent for determination according to the present invention are a one-step method (one-reagent system), the concentration, pH and the like of each component described above may be within the above range. When they are a multi-step method (multi-reagent system), the concentration, pH and the like of each component described above may be defined so that, when the reagents are mixed at a ratio in amount for determining a metal, they may be within the range of concentration, pH and the like for each component described above.

### (8) Sample

According to the present invention, samples to be determined for metals are not particularly limited.

Examples of such samples include biological samples, foods, beverages, drinking water, medicines, reagents, river waters, lake waters, seawater and soils.

Biological samples are not particularly limited, examples of which include human or animal blood, serum, plasma, urine, feces, sperm, cerebrospinal fluid, saliva, perspiration, lacrimal fluid, ascites, amniotic fluid, organs such as the brain, tissues such as the hair, skin, nails, muscles or nerves and cells.

Foods are not particularly limited, examples of which include meat, vegetables, cereals, fruits, fishery products and processed foods.

Beverages are not particularly limited, examples of which include juice, tea, coffee, alcohol and milk.

Medicines are not particularly limited, examples of which include infusion, injectables, powders and tablets.

Samples are preferably in a liquid form and when they are not in a liquid form, it is preferable to render them liquid by carrying out pretreatment such as extraction or solubilization according to known methods.

### (9) Method and Reagent for Colorimetrically Determining Metals

The method and reagent for colorimetrically determining metals in samples using chelating coloring agents according to the invention will specifically be described.

For determining a metal in a sample, for example, the sample is mixed with a reagent containing a masking agent for iron, copper and/or nickel. This mixture of the sample and the reagent is mixed with a reagent containing a chelating coloring agent to cause a color forming reaction between the metal in the sample and the chelating coloring agent. The concentration of the metal in the sample can be determined by, for example, determining absorbance before and after this color formation for a change in absorbance due to the chelate color formation.

Further, more specific description will be made by way of example for colorimetrically determining zinc in a sample by coexistence of bis-tris propane as a masking agent for iron, copper and/or nickel.

For determining zinc in a sample, for example, the sample is mixed with a first reagent containing bis-tris propane. This mixture of the sample and the first reagent is mixed with a second reagent containing a chelating coloring agent (for example, 5-Br-PAPS) to cause a color forming reaction between zinc in the sample and the chelating coloring agent. The concentration of zinc in the sample can be determined by, for example, determining absorbance before and after this color formation for a change in absorbance due to the chelate color formation.

The determination of metals according to the present invention may be carried out either by an end-point assay or rate assay. According to the present invention, however, it is preferable to carry out the determination of metals by an end-point assay.

### (10) Method for Reducing Positive Errors due to Iron, Copper and/or Nickel Contained in Sample

The method for reducing positive errors due to iron, copper or nickel contained in a sample according to the present invention is a method for determination by coexistence or inclusion of a masking agent for iron, copper and/or nickel in a method and reagent for colorimetrically determining a metal using a chelating coloring agent.

In this method and reagent for colorimetrically determining a metal in a sample using a chelating coloring agent, carrying out determination for the metal by coexistence or inclusion of a masking agent for iron, copper and/or nickel can prevent positive errors due to iron, copper or nickel from being introduced into measurements of the metal to be determined, even when the sample includes metals other than the metal to be determined, namely iron, copper or nickel, so that highly accurate measurements of the metal may be obtained.

Also, components of reagents, samples, conditions and the like for carrying out the method for reducing positive errors due to iron, copper or nickel according to the present invention are those as described above.

### EXAMPLES

The present invention will specifically be described below with reference to examples. The present invention is, however, in no way limited to such examples.

### Example 1

Confirming Masking Effects on Iron, Copper and/or Nickel-1

Masking effects on iron, copper and/or nickel were confirmed using bis-tris propane, TAPS, TAPSO, TES, tricine, trishydroxymethylamino methane or bicine as a masking agent for iron, copper and/or nickel.

### 1. Preparation of Reagents

### (1) Preparation of First Reagent A for Determining Zinc

Bis-tris propane was dissolved in pure water to a concentration of 50 mM and its pH was adjusted to 7.0, 8.6 or 9.3 (all at 20°C) to prepare a first reagent A according to the present invention.

### (2) Preparation of First Reagent B for Determining Zinc

TES was dissolved in pure water to a concentration of 50 mM and its pH was adjusted to 7.0, 8.2 or 9.4 (all at 20°C) to prepare a first reagent B according to the present invention.

### (3) Preparation of First Reagent C for Determining Zinc

Trishydroxymethylamino methane was dissolved in pure water to a concentration of 50 mM and its pH was adjusted to 7.0, 8.4 or 9.8 (all at 20°C) to prepare a first reagent C according to the present invention.

### (4) Preparation of First Reagent D for Determining Zinc

TAPS was dissolved in pure water to a concentration of 50 mM and its pH was adjusted to 7.5, 8.6 or 9.5 (all at 20°C) to prepare a first reagent D according to the present invention.

### (5) Preparation of First Reagent E for Determining Zinc

Tricine was dissolved in pure water to a concentration of 50 mM and its pH was adjusted to 8.0, 8.7 or 9.4 (all at 20°C) to prepare a first reagent E according to the present invention.

### (6) Preparation of First Reagent F for Determining Zinc

Bicine was dissolved in pure water to a concentration of 50 mM and its pH was adjusted to 8.4, 8.8 or 9.5 (all at 20°C) to prepare a first reagent F according to the present invention.

### (7) Preparation of Second Reagent for Determining Zinc

The following reagent components were dissolved in pure water to the indicated concentrations to prepare a second reagent.
- 5-Br-PAPS: 0.2 mM
- Triton X-100: 0.05%

### 2. Preparation of Samples

### (1) Preparation of Sample A

1 g/L standard zinc solution (Wako Pure Chemical Industries, Ltd., atomic absorption analysis grade) was diluted with 0.01 N hydrochloric acid to a zinc concentration of 200 µg/dL to provide a sample A.

### (2) Preparation of Sample B

1 g/L standard iron solution (Wako Pure Chemical Industries, Ltd., atomic absorption analysis grade) was diluted with 0.01 N hydrochloric acid to an iron concentration of 200 µg/dL to provide a sample B.

### (3) Preparation of Sample C

1 g/L standard copper solution (Wako Pure Chemical Industries, Ltd., atomic absorption analysis grade) was diluted with 0.01 N hydrochloric acid to a copper concentration of 200 µg/dL to provide a sample C.

### (4) Preparation of Sample D

1 g/L standard nickel solution (Wako Pure Chemical Industries, Ltd., atomic absorption analysis grade) was diluted with 0.01 N hydrochloric acid to a nickel concentration of 200 µg/dL to provide a sample D.

### 3. Determination of Samples

Concentrations of zinc in the samples A to D from 2 above were determined using the first and second reagents for determining zinc prepared in 1 above.

The determination of zinc using the reagents for determination according to the present invention were carried out on a type 7180 automatic analyzer by Hitachi, Ltd. 150 µL of the first reagent A according to the present invention prepared in (1) of 1 above were added to 10 µL each of the samples A to D prepared in 2 above and, after admixing, reaction was allowed at 37°C for five minutes. Then, 50 µL of the second reagent prepared in (7) of 1 above were added and reaction was allowed at 37°C for five minutes. Absorbance was measured at a dominant wavelength of 546 nm and a secondary wavelength of 700 nm, four minutes and 30 seconds after addition of the first reagent (16th point) and 5 minutes and 8 seconds after addition of the second reagent (34th point) to provide the difference.

Then, determination was carried out as described above on a sample having a known zinc concentration and the measured value was compared with the measured value on the four samples described above to provide zinc concentrations in the four samples described above.

Similarly, determinations were carried out replacing the first reagent with the first reagents (B to F) for determining zinc prepared in (2) to (6) of 1 described above.

### 4. Results of Determination

The results of determination on the samples are shown in Table 1.

**Table 1**

| First Reagent A for Determining Zinc | | | |
|---|---|---|---|
| | Measured value of zinc concentration in sample (µg/dL) | | |
| Sample | pH | | |
| | 7.0 | 8.6 | 9.3 |
| A | 200 | 200 | 200 |
| B | 104 | 33 | 18 |
| C | 41 | 11 | 7 |
| D | 55 | 188 | 189 |
| First Reagent B for Determining Zinc | | | |
| | | | |
| Sample | pH | | |
| | 7.0 | 8.2 | 9.4 |
| A | 200 | 200 | 200 |
| B | 36 | 39 | 15 |
| C | 127 | 159 | 162 |
| D | 209 | 193 | 192 |
| First Reagent C for Determining Zinc | | | |
| | Measured value of zinc concentration in sample (µg/dL) | | |
| Sample | pH | | |
| | 7.0 | 8.4 | 9.8 |
| A | 200 | 200 | 200 |
| B | 17 | 20 | 26 |
| C | 124 | 152 | 159 |
| D | 222 | 192 | 194 |
| First Reagent D for Determining Zinc | | | |
| | Measured value of zinc concentration in sample (µg/dL) | | |
| Sample | pH | | |
| | 7.5 | 8.6 | 9.5 |
| A | 200 | 200 | 200 |
| B | 14 | 19 | 21 |
| C | 134 | 163 | 166 |
| D | 196 | 191 | 189 |
| First Reagent E for Determining Zinc | | | |
| | Measured value of zinc concentration in sample (µg/dL) | | |
| Sample | pH | | |
| | 8.0 | 8.7 | 9.4 |
| A | 200 | 200 | 200 |
| B | 76 | 28 | 13 |
| C | 210 | 163 | 160 |
| D | 104 | 75 | 134 |
| First Reagent E for Determining Zinc | | | |
| | Measured value of zinc concentration in sample (µg/dL) | | |
| Sample | pH | | |
| | 8.4 | 8.8 | 9.5 |
| A | 200 | 200 | 200 |
| B | 192 | 96 | 34 |
| C | 149 | 163 | 171 |
| D | 65 | 54 | 63 |

As apparent from Table 1, it can be seen that, when the first reagent A for determining zinc containing bis-tris propane as a masking agent for iron, copper and/or nickel in the first reagent was used, the reagent did not react with the metals other than the metal to be determined (zinc), namely iron and copper. It can also be seen that, for nickel as well, reaction was reduced at pH 7.0.

Further, it can be seen that, when the first reagent B for determining zinc containing TES as a masking agent for iron, copper and/or nickel, the first reagent C for determining zinc containing trishydroxymethylamino methane as the masking agent described above and the first reagent D for determining zinc containing TAPS as the masking agent described above in the first reagent were used, reaction with the metals other than the metal to be determined (zinc), namely iron and copper was reduced, while reaction with nickel occurred. Further, it can be seen that, when the first reagent E for determining zinc containing tricine as a masking agent for iron, copper and/or nickel and the first reagent F for determining zinc containing bicine as the masking agent described above in the first reagent were used, reaction with the metals other than the metal to be determined (zinc), namely iron and nickel were reduced, while reaction with copper occurred.

Based on the above, it has been confirmed that, in a method for determining zinc in a sample using 5-Br-PAPS as a chelating coloring agent, the method for determination and the reagent for determination according to the present invention containing bis-tris propane, TAPS, TES, tricine, trishydroxymethylamino methane or bicine are effective in reducing color formation of 5-Br-PAPS in relation to iron, copper and/or nickel contained in the sample.

Specifically, it has been confirmed that the concentration of the metal to be determined (zinc) can accurately be determined even on a sample in which metals other than the metal to be determined, namely iron, copper and/or nickel are included.

From these results, it has also been confirmed that bis-tris propane is capable of masking all of iron, copper and nickel by adjusting pH in an appropriate manner.

### Example 2

Confirming Masking Effects on Iron, Copper and/or Nickel-2

Masking effects on iron, copper or nickel were confirmed using bis-tris propane, TES or trishydroxymethylamino methane as a masking agent for iron, copper and/or nickel.

### 1. Preparation of Reagents

### (1) Preparation of First Reagent A for Determining Zinc

Bis-tris propane was dissolved in pure water to a concentration of 50 mM and its pH was adjusted to 7.0, 8.6 or 9.3 (all at 20°C) to prepare a first reagent A for determining zinc.

### (2) Preparation of First Reagent B for Determining Zinc

TES was dissolved in pure water to a concentration of 50 mM and its pH was adjusted to 7.0, 7.8 or 8.2 (all at 20°C) to prepare a first reagent B for determining zinc.

### (3) Preparation of First Reagent C for Determining Zinc

Trishydroxymethylamino methane was dissolved in pure water to a concentration of 50 mM and its pH was adjusted to 7.0, 8.4 or 9.8 (all at 20°C) to prepare a first reagent C for determining zinc.

### (4) Preparation of Second Reagent for Determining Zinc

The following reagent components were dissolved in pure water to the indicated concentrations to prepare a second reagent for determining zinc.
- Nitro-PAPS: 0.2 mM
- Triton X-100: 0.05%

### 2. Preparation of Samples

The samples A to D prepared in (1) to (4) of 2 of Example 1 were used unmodified.

### 3. Determination of Samples

Concentrations of zinc in the samples A to D from 2 above were determined using the first and second reagents for determining zinc prepared in 1 above in the same manner as 3 of Example 1.

### 4. Results of Determination

The results of determination on the samples are shown in Table 2.

**Table 2**

| First Reagant A for Determining Zinc | | | |
|---|---|---|---|
| | Measured value of zinc concentration in sample (µg/dL) | | |
| Sample | pH | | |
| | 7.0 | 8.6 | 9.3 |
| A | 200 | 200 | 200 |
| B | 106 | 18 | 3 |
| C | 31 | 7 | 1 |
| D | 53 | 215 | 217 |

| First Reagant B for Determing Zinc | | | |
|---|---|---|---|
| | Measured value of zinc concentration in sample (µg/dL) | | |
| Sample | pH | | |
| | 7.0 | 7.8 | 8.2 |
| A | 200 | 200 | 200 |
| B | 26 | 25 | 24 |
| C | 144 | 164 | 172 |
| D | 235 | 196 | 206 |

| First Reagent C for Determining ZINC | | | |
|---|---|---|---|
| | Measured value of zinc concentration in sample (µg/dL) | | |
| Sample | pH | | |
| | 7.0 | 8.4 | 9.8 |
| A | 200 | 200 | 200 |
| B | 10 | 21 | 21 |
| C | 140 | 160 | 164 |
| D | 267 | 221 | 217 |

As apparent from Table 2, it can be seen that, when the first reagent A for determining zinc containing bis-tris propane as a masking agent for iron, copper and/or nickel in the first reagent was used, the reagent did not react with the metals other than the metal to be determined (zinc), namely iron and copper. It can also be seen that, for nickel as well, reaction was reduced at pH 7.0.

Further, it can be seen that, when the first reagent B for determining zinc containing TES as a masking agent for iron, copper and/or nickel and the first reagent C for determining zinc containing trishydroxymethylamino methane as the masking agent described above in the first reagent were used, reaction with the metals other than the metal to be determined (zinc), namely iron and copper was reduced, while reaction with nickel occurred.

Based on the above, it has been confirmed that, in a method and reagent for determining zinc in a sample using nitro-PAPS, the method for colorimetric determination and the reagent for colorimetric determination according to the present invention containing bis-tris propane, TES or trishydroxymethylamino methane are effective in reducing color formation of nitro-PAPS in relation to iron, copper and/or nickel contained in the sample. Specifically, it has been confirmed that the concentration of the metal to be determined (zinc) can accurately be determined even on a sample in which metals other than the metal to be determined, namely iron, copper and/or nickel are included.

From these results, it has also been confirmed that bis-tris propane is capable of masking all of iron, copper and nickel by adjusting pH in an appropriate manner.

### Example 3

Confirming Masking Effects on Iron, Copper and/or Nickel-3

Masking effects on iron, copper or nickel were confirmed using bis-tris propane as a masking agent for iron, copper and/or nickel.

### 1. Preparation of Reagents for Determination

### (1) Preparation of First Reagent for Determining Zinc

The following reagent components were dissolved in pure water to the indicated concentrations and its pH was adjusted to 9.5 (at 20°C) to prepare eight varieties of a first reagent for determining zinc having different concentrations of Brij-35 (nonionic surface active agent).
- Brij-35: 0%, 0.1%, 0.7%, 1.1%, 1.3%, 1.6%, 2.0% or 6.7%
- Bis-tris propane: 30 mM

### (2) Preparation of Second Reagent for Determining Zinc

The following reagent components were dissolved in pure water to the indicated concentrations to prepare a second reagent for determining zinc.
- 5-Br-PAPS: 0.13 mM
- Triton X-100: 0.05%

### 2. Preparation of Samples

### (1) Preparation of Sample A

1 g/L standard zinc solution (Wako Pure Chemical Industries, Ltd., atomic absorption analysis grade) was diluted with 0.01 N hydrochloric acid to a zinc concentration of 500 µg/dL to provide a sample A.

### (2) Preparation of Sample B

1 g/L standard zinc solution (Wako Pure Chemical Industries, Ltd., atomic absorption analysis grade) was diluted with 0.01 N hydrochloric acid to a zinc concentration of 1,000 µg/dL to provide a sample B.

### (3) Preparation of Sample C

1 g/L standard iron solution (Wako Pure Chemical Industries, Ltd., atomic absorption analysis grade) was diluted with 0.01 N hydrochloric acid to an iron concentration of 200 pg/dL to provide a sample C.

### (4) Preparation of Sample D

1 g/L standard copper solution (Wako Pure Chemical Industries, Ltd., atomic absorption analysis grade) was diluted with 0.01 N hydrochloric acid to a copper concentration of 200 µg/dL to provide a sample D.

### (5) Preparation of Sample E

1 g/L standard nickel solution (Wako Pure Chemical Industries, Ltd., atomic absorption analysis grade) was diluted with 0.01 N hydrochloric acid to a nickel concentration of 200 µg/dL to provide a sample E.

### 3. Determination of Samples

Concentrations of zinc in the samples A to E from 2 above were determined using the eight varieties of the first reagents and the second reagent for determining zinc prepared in 1 above in the same manner as 3 of Example 1.

### 4. Results of Determination

The results of determination on the samples are shown in Table 3.

**Table 3**

| | Measured value of zinc concentration in sample (µg/dL) | | | | | | | |
|---|---|---|---|---|---|---|---|---|
| | First reagent for determining zinc | | | | | | | |
| Sample | Concentration of nonionic surface active agent (%) | | | | | | | |
| | 0 | 0.1 | 0.7 | 1.1 | 1.3 | 1.6 | 2.0 | 6.7 |
| A | 502 | 506 | 506 | 505 | 501 | 504 | 503 | 497 |
| B | 970 | 1,016 | 1,011 | 1,006 | 994 | 995 | 990 | 944 |
| C | 2 | 2 | 0 | 0 | 0 | 1 | 1 | 1 |
| D | 3 | 15 | 6 | 4 | 4 | 3 | 3 | 2 |
| E | 167 | 107 | 45 | 31 | 26 | 24 | 20 | 9 |

As apparent from Table 3, it can be seen that, when the first reagent for determining zinc did not contain Brij-35, the reagent did not react with the metals other than the metal to be determined (zinc), namely iron and copper, but reacted with nickel. It can however be seen that having Brij-35 contained in the first reagent reduced the reaction with nickel.

Based on the above, it has been confirmed that, in a method for determining zinc in a sample using 5-Br-PAPS, when the first reagent for determining zinc according to the present invention containing bis-tris propane as a masking agent for iron, copper or nickel further contained a nonionic surface active agent, the reagent was more effective in reducing color formation of 5-Br-PAPS in relation to nickel contained in the sample than when the reagent did not contain the nonionic surface active agent.

Specifically, it has been confirmed that the concentration of the metal to be determined (zinc) can accurately be determined even on a sample in which metals other than the metal to be determined (zinc), namely iron, copper and/or nickel are included.

### Example 4

Confirming Masking Effects on Iron, Copper and/or Nickel-4

Masking effects on iron, copper and/or nickel were confirmed using bis-tris propane as a masking agent for iron, copper and/or nickel.

### 1. Preparation of Reagents for Determination

### (1) Preparation of First Reagent for Determining Zinc according to the Present Invention

The following reagent components were dissolved in pure water to the indicated concentrations and its pH was adjusted to 9.5 (at 20°C) to prepare seven varieties of a first reagent for determining zinc according to the present invention having different concentrations of bis-tris propane.
- Bis-tris propane: 3 mM, 15 mM, 24 mM, 30 mM, 36 mM, 45 mM or 150 mM
- Brij-35: 1.33%

### (2) Preparation of First Control Reagent for Determining Zinc

Except that bis-tris propane of the first reagent for determining zinc according to the present invention of (1) above was not contained, a first control reagent for determining zinc was prepared in the same manner as (1) above.

### (3) Preparation of Second Reagent for Determining Zinc

The second reagent for determining zinc prepared in (2) of 1 of Example 3 was used unmodified.

### 2. Preparation of Samples

The samples A to E prepared in (1) to (5) of 2 of Example 3 were used unmodified.

### 3. Determination of Samples

Concentrations of zinc in the samples A to E from 2 above were determined using the first and second reagents for determining zinc prepared in 1 above in the same manner as 3 of Example 1.

### 4. Results of Determination

The results of determination on the samples are shown in Table 4.

**Table 4**

| | Measured value of zinc concentration in sample (µg/dL) | | | | | | | |
|---|---|---|---|---|---|---|---|---|
| | First control reagent | First reagent according to present invention | | | | | | |
| Sample | Bis-tris propane concentration (mM) | | | | | | | |
| | 0 | 3 | 15 | 24 | 30 | 36 | 45 | 150 |
| A | 510 | 505 | 508 | 504 | 506 | 505 | 504 | 496 |
| B | 1,038 | 1,017 | 1,015 | 1,006 | 1,007 | 1,002 | 985 | 941 |
| C | 29 | 3 | 1 | 0 | 1 | 0 | 1 | 0 |
| D | 179 | 11 | 5 | 5 | 3 | 2 | 4 | 1 |
| E | 192 | 30 | 27 | 27 | 27 | 27 | 26 | 26 |

As apparent from Table 4, it can be seen that, when the first control reagent for determining zinc not containing bis-tris propane as a masking agent for iron, copper and/or nickel in the first reagent was used, the reagent reacted with all of the metals, zinc, iron, copper and nickel.

In contrast, it can be seen that, when the first reagents for determining zinc according to the present invention containing bis-tris propane as a masking agent for iron, copper and/or nickel in the first reagents were used, the reagents reacted only with zinc as a metal to be determined, but not with iron, copper or nickel.

Based on the above, it has been confirmed that, in a method for determining zinc in a sample using 5-Br-PAPS, when the first reagents for determining zinc according to the present invention containing bis-tris propane as a masking agent for iron, copper and/or nickel were used, the reagents were more effective in reducing color formation of 5-Br-PAPS in relation to iron, copper and/or nickel contained in the sample than when the first control reagent for determining zinc not containing the masking agent for iron, copper and/or nickel was used.

Specifically, it has been confirmed that the concentration of the metal to be determined (zinc) can accurately be determined even on a sample in which metals other than the metal to be determined (zinc), namely iron, copper and/or nickel are included.

### Example 5

### Recovery Test

A study was made on recovery tests for serum samples in the method and reagent for colorimetrically determining zinc according to the present invention.

### 1. Preparation of Reagents for Determination

### (1) Preparation of First Reagent for Determining Zinc

The first reagent prepared in (1) of 1 of Example 3 was used unmodified.

### (2) Preparation of Second Reagent for Determining Zinc

The second reagent prepared in (2) of 1 of Example 3 was used unmodified.

### 2. Samples

1 g/L standard zinc solution (Wako Pure Chemical Industries, Ltd., atomic absorption analysis grade) was diluted with 0.01 N hydrochloric acid and added to a human serum sample to a zinc concentration of 100 µg/dL to provide a sample.

### 3. Determination of Samples

Concentration of zinc in the sample from 2 above was determined using the first and second reagents for determining zinc prepared in 1 above in the same manner as 3 of Example 1. Subsequently, recovery was calculated by subtracting the zinc concentration in the human serum sample before addition of the standard zinc solution from the measurement of zinc obtained and dividing the value by the amount of addition of the standard zinc solution.

Deproteinization was not carried out on the samples in the determination according to the present invention.

The results of determination on the samples are shown in Table 5.

**Table 5**

| | Sample | Concentration of nonionic surface active agent (%) | | | | | | | |
|---|---|---|---|---|---|---|---|---|---|
| | | 0 | 0.1 | 0.7 | 1.1 | 1.3 | 1.6 | 2.0 | 6.7 |
| Measured value of zinc concentration in sample (µg/dL) | Serum only | 61 | 83 | 70 | 69 | 67 | 65 | 65 | 63 |
| | Serum with standard zinc solution added to zinc concentration of 100 µg/dL | 149 | 181 | 169 | 168 | 166 | 167 | 164 | 162 |
| Recove Recovery (%) | | 86.8 | 96.5 | 96.8 | 97.4 | 97.2 | 100.4 | 97.6 | 97.7 |

As apparent from Table 5, it can be seen that, when Brij-35 was not contained in the first reagent, the recovery was only 86.8%, while having Brij-35 contained in the first reagent improved the recovery.

Based on the above, it has been confirmed that, in a method and reagent for determining zinc in a sample using 5-Br-PAPS, when the first reagent for determining zinc containing bis-tris propane as a masking agent for iron, copper and/or nickel further contained a nonionic surface active agent, an excellent average recovery of 97.9% was obtained in comparison with when the nonionic surface active agent was not contained.

Specifically, it has been confirmed that, according to the present invention, the concentration of zinc can accurately be determined without being influenced by components in blood such as proteins even without carrying out deproteinization of a serum sample.

Based on the above, it has been confirmed that the reagent for determining zinc according to the present invention can accurately determine zinc concentrations in samples without needing deproteinization of the samples.

### Example 6

### Comparison with Prior Art Method

Using ICP emission spectrometry as a prior art method and the present invention, zinc concentrations were determined in identical samples to compare measured values.

### 1. Preparation of Reagent for Determination

### (1) Preparation of First Reagent for Determining Zinc

The following reagent components were dissolved in pure water to the indicated concentrations to prepare a first reagent for determining zinc according to the present invention.
- Bis-tris propane: 30 mM
- Brij-35: 1.33%

### (2) Preparation of Second Reagent for Determining Zinc

The second reagent prepared in (2) of 1 of Example 3 was used unmodified.

### 2. Samples

43 varieties of human serum (Sample No. 1 to 43) were provided.

### 3. Determination of Samples

### (1) Determination Using Reagent according to the Present Invention

Concentration of zinc in each human serum sample from 2 above was determined using the first and second reagents for determining zinc prepared in 1 above in the same manner as 3 of Example 1.

Deproteinization of the samples was not carried out in the determination according to the present invention.

### (2) Determination Using Prior Art Method (ICP Emission Spectrometry)

Concentration of zinc in each human serum sample from 2 above was determined using ICPS-8100 (ICP emission spectrometer) from Shimadzu Corporation. For determination using ICP emission spectrometry, deproteinization was carried out in which nitric acid was added to the samples followed by removing proteins through centrifugation.

### 4. Results of Determination

The results of determination on the samples are shown in Table 6. The values presented in Table 6 are average values of two measurements of each sample for the method for determination and the reagent for determination according to the present invention, and are the correction factor for the volume displacement error of one measurement of each sample for ICP emission spectrometry.

**Table 6**

| | Measured value of zinc concentration in sample (µg/dL) | |
|---|---|---|
| Sample No. | Present invention | Prior art method |
| 1 | 38.8 | 37.3 |
| 2 | 89.5 | 90.0 |
| 3 | 73.8 | 72.8 |
| 4 | 76.1 | 75.3 |
| 5 | 59.4 | 57.7 |
| 6 | 71.1 | 67.1 |
| 7 | 76.9 | 70.1 |
| 8 | 83.7 | 82.6 |
| 9 | 51.0 | 52.0 |
| 10 | 94.0 | 92.1 |
| 11 | 69.9 | 66.3 |
| 12 | 74.0 | 70.1 |
| 13 | 76.4 | 75.3 |
| 14 | 90.4 | 84.9 |
| 15 | 64.7 | 66.5 |
| 16 | 60.4 | 59.9 |
| 17 | 79.5 | 77.3 |
| 18 | 81.2 | 78.2 |
| 19 | 74.5 | 72.4 |
| 20 | 85.1 | 83.7 |
| 21 | 84.0 | 82.5 |
| 22 | 61.6 | 60.7 |
| 23 | 56.3 | 55.4 |
| 24 | 77.3 | 76.7 |
| 25 | 72.5 | 71.0 |
| 26 | 70.2 | 69.9 |
| 27 | 68.4 | 68.6 |
| 28 | 63.3 | 62.2 |
| 29 | 68.2 | 65.7 |
| 30 | 107.8 | 102.4 |
| 31 | 68.5 | 65.6 |
| 32 | 82.3 | 78.1 |
| 33 | 72.5 | 69.2 |
| 34 | 78.0 | 77.0 |
| 35 | 132.9 | 131.0 |
| 36 | 83.4 | 84.3 |
| 37 | 73.1 | 74.3 |
| 38 | 89.0 | 87.9 |
| 39 | 111.8 | 113.9 |
| 40 | 82.6 | 80.5 |
| 41 | 71.2 | 68.3 |
| 42 | 94.1 | 91.1 |
| 43 | 95.4 | 91.5 |
| Average | 77.55 | 75.81 |

As apparent from Table 6, it can be seen that the determination results using the reagent for determining zinc according to the present invention correspond well with those from ICP emission spectrometry as a prior art method.

Based on the above, it has been confirmed that, in a method and reagent for determining zinc in a sample using 5-Br-PAPS according to the present invention, the reagent in which bis-tris propane coexists or is contained as a masking agent for iron, copper and/or nickel can accurately determine zinc concentrations in samples without needing deproteinization of the samples.

## Claims

1. A method for colorimetrically determining a metal in a sample using a chelating coloring agent, **characterized by** coexistence of a masking agent for iron, copper and/or nickel.

2. The method for colorimetrically determining a metal in a sample according to Claim 1, wherein the chelating coloring agent is 2-(5-bromo-2-pyridylazo)-5-(N-propyl-N-3-sulfopropylamino)phenol or a salt thereof.

3. The method for colorimetrically determining a metal in a sample according to Claim 1, wherein the chelating coloring agent is 2-(5-nitro-2-pyridylazo)-5-(N-propyl-N-3-sulfopropylamino)phenol or a salt thereof.

4. The method for colorimetrically determining a metal in a sample according to any one of Claims 1 to 3, wherein a surface active agent is used.

5. The method for colorimetrically determining a metal in a sample according to any one of Claims 2 to 4, wherein deproteinization of the sample is unnecessary.

6. The method for colorimetrically determining a metal in a sample according to any one of Claims 1 to 5, wherein the masking agent for iron, copper and/or nickel is one or more selected from bis-tris propane, Bis-Tris, TAPS, TAPSO, TES, tricine, trishydroxymethylamino methane, BES or bicine.

7. The method for colorimetrically determining a metal in a sample according to any one of Claims 1 to 5, wherein the masking agent for iron, copper and/or nickel is a compound represented by the general formula (I): 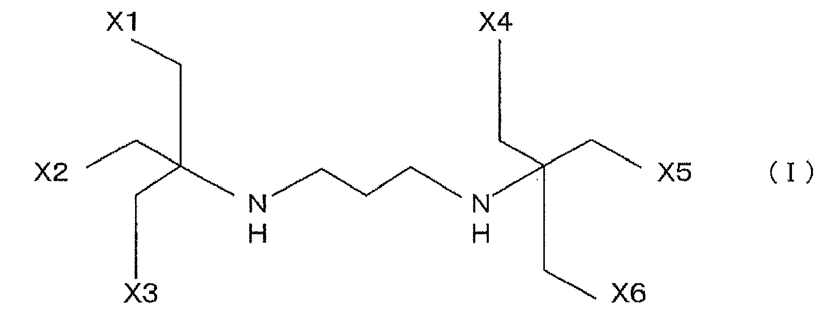 wherein X1 to X6 are respectively a hydrogen atom or an unsubstituted or substituted hydroxyl, carboxyl, SH or amino group.

8. The method for colorimetrically determining a metal in a sample according to any one of Claims 1 to 7, wherein the metal in a sample to be colorimetrically determined is zinc.

9. The method for colorimetrically determining a metal in a sample according to any one of Claims 1 to 8, wherein positive errors due to iron, copper or nickel contained in the sample are reduced.

10. A method for colorimetrically determining zinc in a sample using 2-(5-bromo-2-pyridylazo)-5-(N-propyl-N-3-sulfopropylamino)phenol or a salt thereof, wherein deproteinization of the sample is unnecessary.

11. A reagent for colorimetrically determining a metal in a sample using a chelating coloring agent, **characterized by** inclusion of a masking agent for iron, copper and/or nickel.

12. The reagent for colorimetrically determining a metal in a sample according to Claim 11, wherein the chelating coloring agent is 2-(5-bromo-2-pyridylazo)-5-(N-propyl-N-3-sulfopropylamino)phenol or a salt thereof.

13. The reagent for colorimetrically determining a metal in a sample according to Claim 11, wherein the chelating coloring agent is 2-(5-nitro-2-pyridylazo)-5-(N-propyl-N-3-sulfopropylamino)phenol or a salt thereof.

14. The reagent for colorimetrically determining a metal in a sample according to any one of Claims 11 to 13, further containing a surface active agent.

15. The reagent for colorimetrically determining a metal in a sample according to any one of Claims 12 to 14, wherein deproteinization of the sample is unnecessary.

16. The reagent for colorimetrically determining a metal in a sample according to any one of Claims 11 to 15, wherein the masking agent for iron, copper and/or nickel is one selected from bis-tris propane, TAPS, TAPSO, TES, tricine, trishydroxymethylamino methane or bicine.

17. The reagent for colorimetrically determining a metal in a sample according to any one of Claims 11 to 15, wherein the masking agent for iron, copper and/or nickel is a compound represented by the general formula (I): 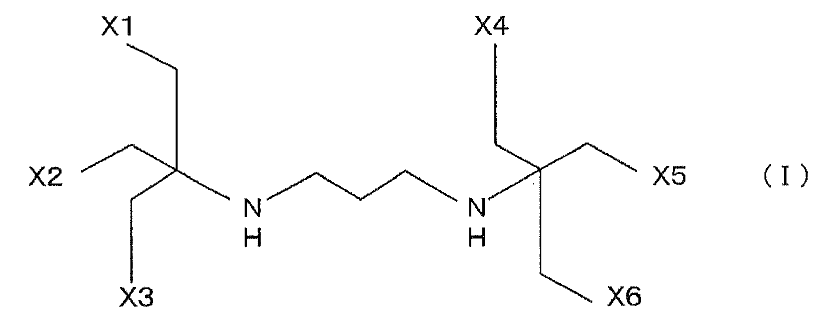 wherein X1 to X6 are respectively a hydrogen atom or an unsubstituted or substituted hydroxyl, carboxyl, SH or amino group.

18. The reagent for colorimetrically determining a metal in a sample according to any one of Claims 11 to 17, wherein positive errors due to iron, copper or nickel contained in the sample are reduced.

19. A masking agent for iron, copper and/or nickel, comprising bis-tris propane, TAPS, TAPSO, TES, tricine, trishydroxymethylamino methane or bicine.

20. A masking agent for iron, copper and/or nickel, comprising a compound represented by the general formula (I) : 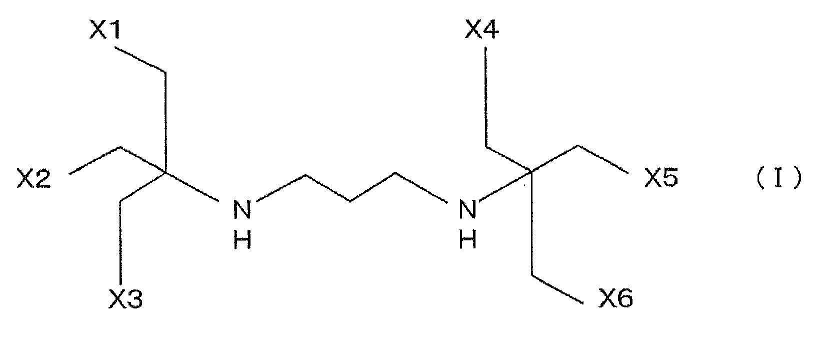 wherein X1 to X6 are respectively a hydrogen atom or an unsubstituted or substituted hydroxyl, carboxyl, SH or amino group.

21. A method for reducing positive errors due to iron, copper or nickel contained in a sample, by coexistence of a masking agent for iron, copper and/or nickel, in a method for colorimetrically determining a metal in the sample using a chelating coloring agent.

22. The method for reducing positive errors due to iron, copper or nickel contained in a sample according to Claim 21, wherein the chelating coloring agent is 2-(5-bromo-2-pyridylazo)-5-(N-propyl-N-3-sulfopropylamino)phenol or a salt thereof.

23. The method for reducing positive errors due to iron, copper or nickel contained in a sample according to Claim 21, wherein the chelating coloring agent is 2-(5-nitro-2-pyridylazo)-5-(N-propyl-N-3-sulfopropylamino)phenol or a salt thereof.

24. The method for reducing positive errors due to iron, copper or nickel contained in a sample according to any one of Claims 21 to 23, wherein the masking agent for iron, copper and/or nickel is one selected from bis-tris propane, TAPS, TAPSO, TES, tricine, trishydroxymethylamino methane or bicine.

25. The method for reducing positive errors due to iron, copper or nickel contained in a sample according to any one of Claims 21 to 24, wherein the masking agent for iron, copper and/or nickel is a compound represented by the general formula (I): 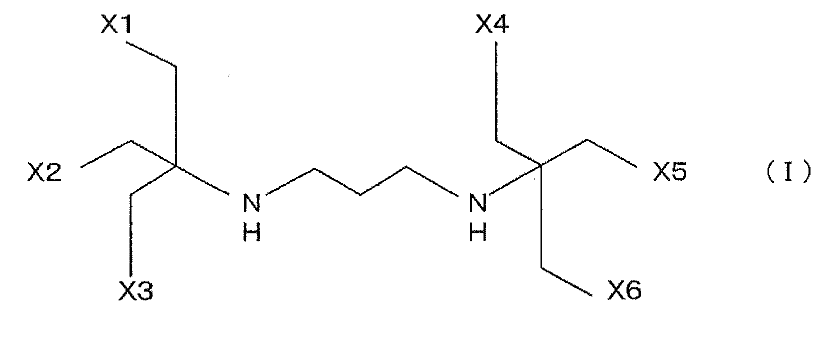 wherein X1 to X6 are respectively a hydrogen atom or an unsubstituted or substituted hydroxyl, carboxyl, SH or amino group.
